# EUROPÄISCHE PATENTANMELDUNG

(11) **EP 3 682 970 A1**
(43) Veröffentlichungstag der Anmeldung: **22.07.2020**
(21) Anmeldenummer: 20154836.9
(22) Anmeldetag: 23.03.2012
(51) Int. Cl.: B01L 3/14, G01N 33/49, A61M 1/34

(54) **VORRICHTUNG ZUM ABTRENNEN EINES ÜBERSTANDS EINER FLÜSSIGEN PROBE**

(30) Priorität: 24.03.2011 EP 11002440
(62) Teilanmeldung aus: 12710096.4
(71) Anmelder: Boehringer Ingelheim Microparts GmbH, 55216 Ingelheim am Rhein (DE)
(72) Erfinder: SCHOEN, Christian, 55216 Ingelheim am Rhein (DE); THIELE, Tanja, 55216 Ingelheim am Rhein (DE)
(74) Vertreter: Weyers, Christopher

(57) **Zusammenfassung**

Es wird eine Vorrichtung zum Abtrennen eines Überstands einer flüssigen Probe vorgeschlagen, wobei die Vorrichtung einen Ablauf für den Überstand und eine dem Ablauf zugeordnete Ventileinrichtung aufweist, wobei sich der Ablauf oberhalb eines Rückhaltebereichs, der in einem unteren Bereich des Aufnahmeraums gebildet ist, in den Aufnahmeraum öffnet, so dass bei geöffneter Ventileinrichtung der Überstand der Probe nur oberhalb des Rückhaltebereichs aus dem Aufnahmeraum abfließt, wobei der Rückhaltebereich von einem Boden des Aufnahmeraums gebildet ist, wobei der Boden im Wesentlichen trichterartig ausgebildet ist, wobei der Ablauf (5) röhrchenartig ausgebildet ist.

## Beschreibung

Die vorliegende Erfindung betrifft eine Vorrichtung zur Abtrennung eines Überstands einer flüssigen Probe gemäß dem Oberbegriff des Anspruchs 1.

Die vorliegende Erfindung befasst sich mit der Manipulation bzw. Aufkonzentrierung einer flüssigen Probe, insbesondere einer Suspension. Hierbei handelt es sich insbesondere um eine biologische Probe oder Probenflüssigkeit, insbesondere um Blut, Urin oder eine sonstige menschliche oder tierische Körperflüssigkeit.

Um feste Bestandteile einer flüssigen Probe aufzukonzentrieren, kann eine Zentrifugation erfolgen. Bisher ist hierbei jedoch nachteilig, dass der Überstand der zentrifugierten Probe manuell, beispielsweise mit einer Pipette, abgetrennt bzw. abgenommen werden muss.

Die US 2005/0123456 A1 offenbart eine Vorrichtung zur Behandlung einer biologischen Probe, die zur Abtrennung eines Überstands einer flüssigen Probe ausgebildet ist. Die Vorrichtung weist auch einen Ablauf oberhalb eines Rückhaltebereiches, der von einem Boden eines Aufnahmeraums gebildet ist, auf. Der Ablauf ist jedoch nicht röhrchenartig ausgebildet und der an den Ablauf angrenzende Boden ist nicht trichterartig ausgebildet.

Die US 2002/0046614 A1 offenbart ein Gerät zum Testen einer Probe. Es ist ein längliches Gefäß offenbart, bei dem der Boden konisch verjüngt ist. Es sind jedoch kein röhrchenartiger Ablauf und kein trichterartiger Boden offenbart.

Die US 4,722,792 zeigt ein Gefäß zur Zentrifugation einer Probe. Es ist ein oberhalb eines Rückhaltebereiches angeordneter Ablauf gezeigt, der jedoch nicht röhrchenartig ausgebildet ist.

Die US 5,647,990 zeigt ein Gefäß zur Zentrifugation einer Probe. Das Gefäß weist einen Ablauf oberhalb eines Rückhaltebereiches auf, wobei der Ablauf jedoch nicht röhrchenartig ausgebildet ist.

Der vorliegenden Erfindung liegt die Aufgabe zugrunde, eine Vorrichtung zur Abtrennung eines Überstands einer flüssigen Probe anzugeben, wobei eine vereinfachte, insbesondere automatisierte bzw. selbsttätige Manipulation der flüssigen Probe ermöglicht oder erleichtert wird.

Die obige Aufgabe wird durch eine Vorrichtung gemäß Anspruch 1 gelöst. Vorteilhafte Weiterbildungen sind Gegenstand der Unteransprüche.

Ein Aspekt der vorliegenden Erfindung liegt darin, dass zur Abtrennung eines Überstands einer flüssigen Probe ein Aufnahmeraum mit einem Ablauf vorgesehen wird, wobei dem Ablauf eine Ventileinrichtung zugeordnet wird. So kann das Ablaufen bzw. Abtrennen des Überstands der flüssigen Probe gezielt bzw. gesteuert erfolgen. Insbesondere kann zunächst eine Zentrifugation der Probe erfolgen und anschließend durch Öffnen der Ventileinrichtung der Überstand aus dem Aufnahmeraum abgeleitet und damit abgetrennt werden. Dies gestattet eine sehr einfache Manipulation der flüssigen Probe. Insbesondere ist der Einsatz einer Pipette oder dgl. zum Abtrennen des Überstands nicht erforderlich.

Die Ventileinrichtung öffnet vorzugsweise selbsttätig und/oder durch Auflösen einer Membran und/oder durch Bersten bei Erreichen oder Überschreiten eines bestimmten Drucks. Dies vereinfacht die Handhabung wesentlich.

Gemäß einem anderen Aspekt der vorliegenden Erfindung wird die Probe in dem Aufnahmeraum zentrifugiert und anschließend - insbesondere durch Zentrifugal- und/oder Gravitationskraft - aus dem Aufnahmeraum über einen Ablauf abgeleitet, der temporär verschlossen ist und/oder selbsttätig öffnet. Dies gestattet einen sehr einfachen Aufbau und ermöglicht eine einfache Manipulation der flüssigen Probe.

Ein weiterer Aspekt der vorliegenden Erfindung liegt darin, eine Ventileinrichtung mit einer löslichen Membran zum temporären Zurückhalten einer Flüssigkeit in einem Aufnahmeraum bzw. für eine normalerweise geschlossene Ventileinrichtung einzusetzen. Die Ventileinrichtung öffnet durch Anlösen oder Auflösen der Membran durch die Flüssigkeit selbsttätig. Dies ermöglicht einen sehr einfachen Aufbau und gestattet eine sehr einfache Manipulation bzw. Handhabung.

Die vorliegende Erfindung befasst sich insbesondere mit der Aufkonzentrierung fester Bestandteile der flüssigen Probe. Die aufkonzentrierten Bestandteile können dann einer weiteren Untersuchung zugeführt werden während der Überstand abgetrennt bzw. abgeleitet wird und insbesondere verworfen werden kann. Die vorschlagsgemäße Ventileinrichtung kann jedoch grundsätzlich auch für sonstige Zwecke eingesetzt werden.

Die vorschlagsgemäßen Lösungen ermöglichen es insbesondere, den Überstand ohne manuelles Eingreifen und ohne Interaktion von außen abzuleiten bzw. abzutrennen. Dementsprechend wird der Verfahrensablauf bzw. die Handhabung vereinfacht.

Die vorgenannten Aspekte der vorliegenden Erfindung sowie die nachfolgend erläuterten Aspekte der vorliegenden Erfindung können in beliebiger Kombination, aber auch unabhängig voneinander realisiert werden.

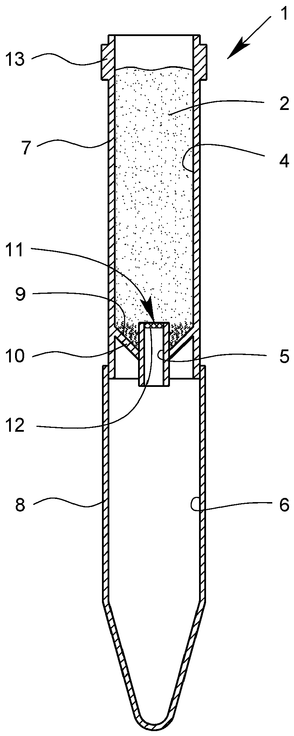

Weitere Vorteile, Merkmale, Eigenschaften und Aspekte der vorliegenden Erfindung ergeben sich aus den Ansprüchen und der folgenden Beschreibung einer bevorzugten Ausführungsform anhand der Zeichnung. Die einzige Figur zeigt:
einen schematischen Schnitt einer vorschlagsgemäßen Vorrichtung.

Die Figur zeigt in einem schematischen Schnitt eine vorschlagsgemäße Vorrichtung 1 zur Manipulation einer flüssigen Probe 2. Insbesondere handelt es sich bei der Probe 2 um eine partikelhaltige Lösung bzw. Suspension mit insbesondere festen Bestandteilen 3. Besonders bevorzugt handelt es sich um eine biologische Probe oder Flüssigkeit, beispielsweise um Blut, Urin oder eine sonstige menschliche oder natürliche Körperflüssigkeit. Jedoch können auch sonstige Flüssigkeiten bzw. Suspensionen oder dgl. als Probe 2 manipuliert werden.

Die Vorrichtung 1 dient besonders bevorzugt der Aufkonzentration von festen bzw. nicht lösbaren Bestandteilen 3 der Probe 2 und/oder der Abtrennung eines Überstands der Probe 2, insbesondere also der überstehenden Flüssigkeit nach Aufkonzentration der Bestandteile 3.

Die Vorrichtung 1 wird insbesondere zur Zentrifugation eingesetzt. Besonders bevorzugt ist die Vorrichtung 1 in eine nicht dargestellte Zentrifuge einsetzbar bzw. zentrifugierbar.

Beim Darstellungsbeispiel ist bereits eine Aufkonzentration der Bestandteile 3 in einem unteren Bereich erfolgt. Die Figur zeigt also insbesondere einen Zustand nach einer Zentrifugation. Der Überstand an Flüssigkeit bzw. der Probe 2 wurde jedoch noch nicht abgetrennt oder abgeleitet.

Die Vorrichtung 1 weist vorzugsweise einen Aufnahmeraum 4 zur Aufnahme der Probe 2 und einen zugeordneten Ablauf 5 zur Ableitung, insbesondere eines Überstands der Flüssigkeit bzw. Probe 2, auf. Die Vorrichtung 1 kann ferner einen Auffangraum 6 aufweisen oder bilden, um den durch den Ablauf 5 abgeleiteten Überstand bzw. durch den Ablauf 5 abgeleitete Flüssigkeit oder dgl. aufzufangen.

Beim Darstellungsbeispiel ist der Aufnahmeraum 4 vorzugsweise in einem Oberteil 7 der Vorrichtung 1 und der Auffangraum 6 vorzugsweise in einem Unterteil 8 der Vorrichtung 1 gebildet. Das Oberteil 7 und das Unterteil 8 sind vorzugsweise als getrennte Bauteile ausgebildet.

Das Unterteil 7 und das Oberteil 8 sind vorzugsweise lösbar miteinander verbindbar. Das Oberteil 7 und das Unterteil 8 sind vorzugsweise kraftschlüssig oder formschlüssig miteinander verbindbar, insbesondere durch Aufstecken, durch Verschrauben, durch einen Bajonettverschluss, durch eine Schnappverbindung oder dgl. Insbesondere ist das Unterteil 8 auf der Oberteil 7 aufsteckbar oder umgekehrt. In der Darstellung gemäß der einzigen Figur ist das Oberteil 7 mit dem Unterteil 8 verbunden.

Das Oberteil 7 und das Unterteil 8 kann auch von einem gemeinsamen Bauteil gebildet sein. Das Unterteil 8 kann in diesem Fall beispielsweise über eine Sollbruchstelle ggf. von dem Oberteil 7 (vollständig) trennbar bzw. lösbar sein. Jedoch ist es auch möglich, dass das Oberteil 7 und das Unterteil 8 nicht voneinander (vollständig) trennbar bzw. lösbar sind.

Insbesondere ist das Unterteil 8 derart am Oberteil 7 gehalten bzw. gesichert, dass sich das Unterteil 8 bei der Zentrifugation der Probe 2 bzw. Vorrichtung 1 nicht vom Oberteil 7 löst.

Die Vorrichtung 1 ist vorzugsweise zumindest im Wesentlichen länglich oder röhrchenartig und/oder hohlzylindrisch ausgebildet. Jedoch sind auch andere Ausgestaltungen möglich.

Der Ablauf 5 ist vorzugsweise in einem gegenüberliegenden Endbereich bzw. in einem Bodenbereich und/oder in einem dem Unterteil 8 benachbarten Bereich angeordnet.

Die Vorrichtung 1 weist vorzugsweise einen Rückhaltebereich 9 auf, der in dem Aufnahmeraum 4 bzw. Oberteil 7 gebildet ist und insbesondere der Aufnahme eines Rückstands der Probe 2, insbesondere der aufkonzentrierten Probe 2 bzw. Bestandteile 3 dient, wie in der Figur schematisch angedeutet. Der Rückhaltebereich 9 wird vorzugsweise von einem stirnwandseitigen Abschnitt bzw. Boden 10 des Aufnahmeraums 4 bzw. Oberteils 7 gebildet.

Der Boden 10 ist beim Darstellungsbeispiel vorzugsweise im Wesentlichen trichterartig oder kegelförmig ausgebildet.

Beim Darstellungsbeispiel ist der Boden 10 vorzugsweise einstückig mit dem Oberteil 7 ausgebildet. Vorzugsweise ist der Ablauf 5 einstückig mit dem Oberteil 7 bzw. Boden 10 ausgebildet.

Der Ablauf 5 erstreckt sich vorzugsweise derart in den Aufnahmeraum 4, insbesondere über den Boden 10 bzw. Rückhaltebereich 9 hinaus in den Aufnahmeraum 4, so dass bei geöffneten Ablauf 5 nur Flüssigkeit oder Überstand oberhalb des Rückhaltebereichs 9 aus dem Aufnahmeraum 4 durch den Ablauf 5 abgeleitet wird. Der Ablauf 5 öffnet sich also oberhalb des Bodens 10 in den Aufnahmeraum 4.

Der Ablauf 5 ist beim Darstellungsbeispiel vorzugsweise röhrchenartig oder hohlzylindrisch ausgebildet. Der Ablauf 5 erstreckt sich beim Darstellungsbeispiel vorzugsweise in die Haupterstreckungsrichtung der Vorrichtung 1.

Beim Darstellungsbeispiel ist der Aufnahmeraum 4 bzw. das Oberteil 7 vorzugsweise oben bzw. an den dem Ablauf 5 oder Boden 10 entfernten Ende offen zur Aufnahme der Probe 2 ausgebildet.

Es ist anzumerken, dass die Darstellung eine bevorzugte Ausrichtung der Vorrichtung 1 wiedergibt, bei der das Oberteil 7 bzw. der Aufnahmeraum 4 oben liegt, wobei der Rückhaltebereich 9 unten liegt und der Ablauf 5 ebenfalls unten angeordnet ist. Diese Ausrichtung der Vorrichtung 1 gilt insbesondere dann, wenn eine Ableitung der Flüssigkeit bzw. des Überstands durch den Ablauf 5 durch Gravitationskraft erfolgt bzw. erfolgen soll. Jedoch kann die Vorrichtung 1 grundsätzlich auch jede sonstige geeignete Ausrichtung haben, insbesondere in Abhängigkeit von der Richtung einer wirkenden Zentrifugalkraft, besonders bevorzugt wenn das Ableiten des Überstands bzw. von Flüssigkeit aus dem Aufnahmeraum 4 durch den Ablauf 5 durch Zentrifugalkraft oder (auch) unter deren Einwirkung erfolgt oder erfolgen soll.

Das Oberteil 7 bzw. Unterteil 8 ist vorzugsweise aus Kunststoff und/oder als Spritzgussteil hergestellt.

Die Vorrichtung 1 weist vorzugsweise eine Ventileinrichtung 11 auf, die insbesondere dem Ablauf 5 zugeordnet ist. Die Ventileinrichtung 11 verschließt den Ablauf 5 vorzugsweise temporär bzw. normalerweise.

Die Ventileinrichtung 11 ist vorzugsweise derart ausgebildet, dass sie selbsttätig und/oder nach einer vorbestimmten Zeit, insbesondere nach Einfüllen oder Aufnehmen der Probe 2 in den Aufnahmeraum 4, öffnet.

Die Ventileinrichtung 11 weist beim Darstellungsbeispiel vorzugsweise eine Membran 12 auf oder ist daraus gebildet. Die Membran 12 ist vorzugsweise löslich, insbesondere wasserlöslich. Besonders bevorzugt wird die Membran 12 durch die Probe 2 gelöst, angelöst oder angegriffen. So kann erreicht werden, dass die Ventileinrichtung 11 bzw. Membran 12 sich öffnet bzw. den Ablauf 5 öffnet oder freigibt.

Die Zeit bis um Öffnen bzw. Freigeben beträgt vorzugsweise einige Minuten, insbesondere mehr als 10 Minuten, besonders bevorzugt mehr als 20 Minuten. So bleibt genügend Zeit, um beispielsweise zunächst nach dem Einfüllen der Probe 2 in den Aufnahmeraum 4 die Vorrichtung 1 bzw. Probe 2 zu zentrifugieren. Das Öffnen der Ventileinrichtung 11 bzw. der Membran 12 erfolgt dann je nach Bedarf noch während der Zentrifugation oder danach, um dann den Überstand der Probe 2, also die Flüssigkeit oberhalb der Öffnung des Ablaufs 5 abzutrennen bzw. aus dem Aufnahmeraum 4 abzuleiten, insbesondere in den Auffangraum 6.

Die Membran 12 ist vorzugsweise relativ dickwandig ausgebildet, um den beim Zentrifugieren auftretenden Belastungen zu widerstehen.

Alternativ oder zusätzlich kann die Ventileinrichtung 11 bzw. Membran 12 auch derart ausgebildet sein, dass sie bei Erreichen oder Überschreiten eines bestimmten Drucks und/oder einer darauf wirkenden Kraft, insbesondere Zentrifugalkraft, öffnet, bricht oder berstet. So kann beispielsweise ein Öffnen der Ventileinrichtung 11 bzw. die Membran 12 und damit des Ablaufs 5 auch dadurch erreicht werden, dass nach einer ersten Zentrifugation bzw. nach ausreichendem Zentrifugieren die Zentrifugalgeschwindigkeit bzw. Drehzahl so stark oder weiter erhöht wird, dass die Ventileinrichtung 11 bzw. die Membran 12 bricht oder berstet oder auf sonstige Weise öffnet. Die Zentrifugation kann also beispielsweise in zwei Stufen bzw. mit zwei Geschwindigkeiten erfolgen. Alternativ oder zusätzlich kann die Geschwindigkeit bzw. Drehzahl auch kontinuierlich oder rampenartig oder in sonstiger Weise erhöht werden.

Der Überstand bzw. die sich oberhalb der Öffnung des Ablaufs 5 in dem Aufnahmeraum 4 befindende Flüssigkeit wird nach Öffnen der Ventileinrichtung 11 bzw. Membran 12 vorzugsweise durch Zentrifugalkraft und/oder Gravitationskraft aus dem Aufnahmeraum 4 durch den Ablauf 5 hindurch abgeleitet.

Der abgeleitete Überstand bzw. die abgeleitete Flüssigkeit wird vorzugsweise in dem Aufnahmeraum 6 des Unterteils 8 aufgefangen bzw. aufgenommen.

Nach der Ableitung des Überstands kann die aufkonzentrierte Probe 2, die im Rückhaltebereich 9 verblieben ist, weiterverwendet werden, beispielsweise für Untersuchungen oder sonstige Manipulationen. Um die Entnahme der aufkonzentrierten Probe 2 bzw. aufkonzentrierten Bestandteile 3 zu erleichtern, kann das Unterteil 8 bedarfsweise von dem Oberteil 7 gelöst werden. Alternativ oder zusätzlich kann die Entnahme auch über die vorzugsweise obenliegende Befüllöffnung des Aufnahmeraums 4 bzw. Oberteils 7 entnommen werden, wobei diese ggf. zuerst wieder geöffnet werden muss, wenn sie für die Zentrifugation beispielsweise durch einen nicht dargstellten Stopfen, Verschluss oder dgl. verschlossen worden ist.

Das Volumen des Rückhaltebereichs 9 bzw. der im Auffangraum 4 zurückgehaltenen bzw. aufkonzentrierten Probe 2 kann durch die Länge des Ablaufröhrchens bzw. die Höhe der Öffnung des Ablaufs 5 im Aufnahmeraum 4 eingestellt oder festgelegt werden.

Die Ventileinrichtung 11 bzw. Membran 12 verschließt den Ablauf 5 vorzugsweise stopfenartig und/oder vollflächig und/oder zumindest flüssigkeitsdicht.

Die Ventileinrichtung 11 bzw. Membran 12 ist vorzugsweise am aufnahmeraumseitigen bzw. einlassseitigen Ende des insbesondere röhrenartig ausgebildeten Ablaufs 5 angeordnet. Jedoch kann die Ventileinrichtung 11 bzw. Membran 12 alternativ oder zusätzlich auch am anderen Ende bzw. auslaufseitigen Ende des Ablaufs 5 und/oder dazwischen den Enden angeordnet sein.

Alternativ kann der Ablauf 5 durch die Ventileinrichtung 11 bzw. ein insbesondere wasserlösliches Material zumindest im Wesentlichen vollständig gefüllt sein.

Bedarfsweise können auch mehrer Membranen 12 eingesetzt werden. Alternativ oder zusätzlich kann auch eine Ventileinrichtung 11 bzw. Membran 12 an jedem Ende des Ablaufs 5 angeordnet sein.

Die Ventileinrichtung 11 bzw. Membran 12 öffnet vorzugsweise ohne manuelle Fremdeinwirkung oder ohne äußere Einwirkungen, insbesondere nach einer bestimmten Zeit oder überschreiten einer bestimmten Zeit, besonders bevorzugt dadurch, dass die flüssige Probe 2 oder ein Bestandteil, insbesondere Wasser, die Membran 12 oder ein sonstiges Teil der Ventileinrichtung 11 angreift, zerstört, auflöst, anlöst und/oder in sonstiger Weise modifiziert.

Die Membran 12 enthält vorzugsweise ein wasserlösliches Material. Insbesondere ist die Membran 12 zumindest im Wesentlichen oder ausschließlich aus einem wasserlöslichen Material hergestellt.

Die Ventileinrichtung 11 bzw. Membran 12 kann beispielsweise durch Kleben, Schweißen, Klemmen, Einpressen und/oder auf jede sonstige Art und Weise mit dem Ablauf 5 verbunden oder in diesen eingesetzt oder eingebracht werden.

Die vorschlagsgemäße Ventileinrichtung 11 bzw. insbesondere wasserlösliche Membran 12 kann grundsätzlich auch für sonstige Ventilzwecke bzw. zum temporären Zurückhalten einer Flüssigkeit 2 insbesondere in einem Aufnahmeraum 4 eingesetzt oder verwendet werden. Die Ventileinrichtung 11 bzw. Membran 12 öffnet dann insbesondere durch zumindest teilweises An- oder Auflösen des Materials bzw. der Membran 12 durch die Flüssigkeit 2 oder einen Bestandteil der Flüssigkeit 2 selbsttätig.

Die Vorrichtung 1 bzw. deren Oberteil 7 weist beim Darstellungsbeispiel vorzugsweise mindestens ein Widerlager 13, insbesondere ein oder mehrere seitliche Vorsprünge oder dgl., zur Halterung in einer (nicht dargestellten) Zentrifuge oder dgl. auf.

Weitere Aspekte der vorliegenden Erfindung, die auch mit den voranstehend beschriebenen Aspekten kombinierbar sind, sind:
1. Vorrichtung 1 zur Abtrennung eines Überstands einer flüssigen Probe 2, mit einem Aufnahmeraum 4 für die Probe 2,
   dadurch gekennzeichnet,
   dass die Vorrichtung 1 einen Ablauf 5 für den Überstand und eine dem Ablauf 5 zugeordnete Ventileinrichtung 11 aufweist.
2. Vorrichtung nach Aspekt 1, dadurch gekennzeichnet, dass sich der Ablauf 5 oberhalb eines Rückhaltebereichs 9, der in einem unteren Bereich des Aufnahmeraum 4 gebildet ist, in den Aufnahmeraum 4 öffnet, so dass bei geöffneter Ventileinrichtung 11 der Überstand der Probe 2 nur oberhalb des Rückhaltebereichs 9 aus dem Aufnahmeraum 4 abfließt.
3. Vorrichtung nach Aspekt 1 oder 2, dadurch gekennzeichnet, dass die Ventileinrichtung 11 selbsttätig öffnend ausgebildet ist.
4. Vorrichtung nach einem der voranstehenden Aspekte, dadurch gekennzeichnet, dass die Ventileinrichtung 11 eine wasserlösliche Membran 12 aufweist oder daraus gebildet ist.
5. Vorrichtung nach einem der voranstehenden Aspekte, dadurch gekennzeichnet, dass die Ventileinrichtung 11 bei Überschreiten eines vorbestimmten Drucks und/oder nach einer vorbestimmten Zeit öffnet oder berstet.
6. Vorrichtung nach einem der voranstehenden Aspekte, dadurch gekennzeichnet, dass die Vorrichtung 1 einen Auffangraum 6 für den Überstand aufweist.
7. Vorrichtung nach Aspekt 6, dadurch gekennzeichnet, dass die Vorrichtung 1 ein Oberteil 7 und ein Unterteil 8 aufweist, wobei der Aufnahmeraum 4 im Oberteil 7 und der Auffangraum 6 im Unterteil 8 gebildet oder angeordnet ist, insbesondere wobei das Oberteil 7 und das Unterteil 8 voneinander lösbar sind und/oder lösbar miteinander verbindbar sind.
8. Vorrichtung nach einem der voranstehenden Aspekte, dadurch gekennzeichnet, dass die Vorrichtung 1 mindestens ein Widerlager 13 zur Halterung in einer Zentrifuge aufweist.
9. Verfahren zum Abtrennen eines Überstands einer flüssigen Probe 2, wobei die Probe 2 in einem Aufnahmeraum 4 zentrifugiert und anschließend der Überstand aus dem Aufnahmeraum 4 entfernt wird,
   dadurch gekennzeichnet,
   dass der Überstand über einen Ablauf 5 - insbesondere durch Zentrifugal- und/oder Gravitationskraft - aus dem Aufnahmeraum 4 abgeleitet wird, wobei ein Ablauf 5 temporär verschlossen ist und/oder selbsttätig geöffnet wird.
10. Verfahren nach Aspekt 9, dadurch gekennzeichnet, dass beim Ableiten des Überstands ein Rückstand der Probe 2 in dem Aufnahmeraum 4 verbleibt, insbesondere da sich der Ablauf 5 oberhalb eines Rückhaltebereichs 9, der in einem unteren Bereich des Aufnahmeraum 4 gebildet ist, in den Aufnahmeraum 4 öffnet, so dass bei geöffnetem Ablauf 5 der Überstand der Probe 2 nur oberhalb des Rückhaltebereichs 9 aus dem Aufnahmebereich 4 abfließt.
11. Verfahren nach Aspekt 9 oder 10, dadurch gekennzeichnet, dass der Ablauf 5 selbsttätig öffnet.
12. Verfahren nach einem der Aspekte 9 bis 11, dadurch gekennzeichnet, dass der Ablauf 5 durch An- oder Auflösen einer löslichen Membran 12 öffnet.
13. Verfahren nach einem der Aspekte 9 bis 12, dadurch gekennzeichnet, dass der Ablauf 5 durch Erhöhen des wirkenden Drucks oder der auf die Probe 2 wirkenden Zentrifugalkraft und/oder nach einer vorbestimmten Zeit öffnet.
14. Verfahren nach einem der Aspekte 9 bis 13, dadurch gekennzeichnet, dass der Überstand in einen dem Aufnahmeraum 4 zugeordneten Auffangraum 6 abgeleitet wird, der vorzugsweise von dem Aufnahmeraum 4 trennbar ist.
15. Verwendung einer Ventileinrichtung 11 mit einer löslichen Membran 12 zum temporären Zurückhalten einer Flüssigkeit 2 in einem Aufnahmeraum 4, wobei die Ventileinrichtung 11 durch An- oder Auflösen der Membran 12 durch die Flüssigkeit 4 selbsttätig öffnet.

### Bezugszeichenliste:

- 1: Vorrichtung
- 2: Probe
- 3: Bestandteil
- 4: Aufnahmeraum
- 5: Ablauf
- 6: Auffangraum
- 7: Oberteil
- 8: Unterteil
- 9: Rückhaltebereich
- 10: Boden
- 11: Ventileinrichtung
- 12: Membran
- 13: Widerlager

## Patentansprüche

1. Vorrichtung (1) zur Abtrennung eines Überstands einer flüssigen Probe (2), mit einem Aufnahmeraum (4) für die Probe (2),
wobei die Vorrichtung (1) einen Ablauf (5) für den Überstand und eine dem Ablauf (5) zugeordnete Ventileinrichtung (11) aufweist,
wobei sich der Ablauf (5) oberhalb eines Rückhaltebereichs (9), der in einem unteren Bereich des Aufnahmeraums (4) gebildet ist, in den Aufnahmeraum (4) öffnet, so dass bei geöffneter Ventileinrichtung (11) der Überstand der Probe (2) nur oberhalb des Rückhaltebereichs (9) aus dem Aufnahmeraum (4) abfließt,
wobei der Rückhaltebereich (9) von einem Boden (10) des Aufnahmeraums (4) gebildet ist, wobei der Boden (10) im Wesentlichen trichterartig ausgebildet ist,
**dadurch gekennzeichnet,**
**dass** der Ablauf (5) röhrchenartig ausgebildet ist.

2. Vorrichtung nach Anspruch 1, **dadurch gekennzeichnet, dass** die Ventileinrichtung (11) selbsttätig öffnend ausgebildet ist.

3. Vorrichtung nach einem der voranstehenden Ansprüche, **dadurch gekennzeichnet, dass** die Ventileinrichtung (11) eine wasserlösliche Membran (12) aufweist oder daraus gebildet ist.

4. Vorrichtung nach einem der voranstehenden Ansprüche, **dadurch gekennzeichnet, dass** die Ventileinrichtung (11) bei Überschreiten eines vorbestimmten Drucks und/oder nach einer vorbestimmten Zeit öffnet oder berstet.

5. Vorrichtung nach einem der voranstehenden Ansprüche, **dadurch gekennzeichnet, dass** die Vorrichtung (1) einen Auffangraum (6) für den Überstand aufweist.

6. Vorrichtung nach Anspruch 5, **dadurch gekennzeichnet, dass** die Vorrichtung (1) ein Oberteil (7) und ein Unterteil (8) aufweist, wobei der Aufnahmeraum (4) im Oberteil (7) und der Auffangraum (6) im Unterteil (8) gebildet oder angeordnet ist, insbesondere wobei das Oberteil (7) und das Unterteil (8) voneinander lösbar sind und/oder lösbar miteinander verbindbar sind.

7. Vorrichtung nach einem der voranstehenden Ansprüche, **dadurch gekennzeichnet, dass** die Vorrichtung (1) mindestens ein Widerlager (13) zur Halterung in einer Zentrifuge aufweist.

8. Vorrichtung nach einem der voranstehenden Ansprüche, **dadurch gekennzeichnet, dass** es sich bei der Probe (2) um eine Suspension mit insbesondere festen Bestandteilen (3) handelt, insbesondere um eine biologische Probe (2), besonders bevorzugt Blut oder Urin oder eine menschliche oder natürliche Körperflüssigkeit.

9. Vorrichtung nach einem der voranstehenden Ansprüche, **dadurch gekennzeichnet, dass** die Ventileinrichtung (11) den Ablauf flüssigkeitsdicht und/oder temporär verschließt.
